# FASCICULE DE BREVET EUROPEEN

(11) **EP 0 934 116 B1**
(45) Date de publication et mention de la délivrance du brevet: **17.09.2003**
(21) Numéro de dépôt: 97910444.5
(22) Date de dépôt: 09.10.1997
(51) Int. Cl.: B01J 29/90, B01J 29/04, C07D 301/12, B01J 38/12

(54) **SYNTHESE D'EPOXYDE COMRENANT LA REGENERATION DU CATALYSEUR DE TYPE SILICALITE AU TITANE**
EPOXYSYNTHESE MIT REGENERIERUNG DER TITANSILIKALITKATALYSATOREN
EPOXIDE SYNTHESIS WITH REGENERATION OF TITANIUM SILICALITE CATALYSTS

(30) Priorité: 25.10.1996 BE 9600911
(43) Date de publication de la demande: 11.08.1999
(73) Titulaire: SOLVAY (Société Anonyme), 1050 Bruxelles (BE)
(72) Inventeur: CATINAT, Jean-Pierre, B-7131 Waudrez (BE); STREBELLE, Michel, B-1150 Bruxelles (BE)
(74) Mandataire: Vande Gucht, Anne
(86) Numéro de dépôt international: EP9705685
(87) Numéro de publication internationale: WO98018556

(56) Documents cités:
- EP-A- 0 200 260
- EP-A- 0 604 689
- EP-A- 0 659 685
- DE-A- 4 425 672

## Description

La présente invention a pour objet un procédé de régénération de catalyseurs de type silicalite au titane, catalyseurs utilisés notamment dans des réactions entre un composé peroxydé, en particulier du peroxyde d'hydrogène, et un co-réactif organique.

Il est connu d'utiliser une silicalite au titane comme catalyseur, notamment dans des réactions d'oxydation d'hydrocarbures saturés pour former des alcools ou des cétones, comme décrit dans la demande de brevet européen EP-A-376453 ou dans des réactions d'époxydation d'oléfines, comme décrit dans la demande de brevet EP-A-100119 ou encore dans des réactions d'hydroxylation de composés aromatiques comme signalé dans la demande EP-A-200260.

L'activité de ces catalyseurs chute cependant rapidement. Il apparaît dès lors essentiel de disposer d'un moyen pour les régénérer afin de pouvoir les utiliser de manière répétée.

La demande de brevet JP 03/114536 décrit un procédé de régénération de catalyseurs de type silicalite au titane par calcination sous air à une température de 400 à 500 °C. Il y est précisé qu'une calcination à une température de moins de 400 °C est insuffisante pour récupérer l'activité catalytique initiale du catalyseur

La présente invention a pour but de procurer un procédé de régénération de catalyseurs de type silicalite au titane qui soit plus efficace que le procédé connu et qui puisse dès lors être mis en oeuvre à plus basse température

En conséquence, l'invention concerne un procédé de régénération d'un catalyseur de type silicalite au titane comprenant un traitement thermique, qui se caractérise en ce que, durant le traitement thermique, on balaie le catalyseur, à une température d'au moins 130 °C, mais inférieure à 400°C, par un courant gazeux dont le temps de séjour massique sur le catalyseur est d'au moins une minute et ne dépasse pas 1 heure.

Par définition, le temps de séjour massique du courant gazeux sur le catalyseur est le rapport entre le poids de catalyseur à régénérer et le débit massique du courant gazeux. Un temps de séjour massique de 2 à 30 minutes s'est avéré particulièrement avantageux.

Le courant gazeux balayant le catalyseur peut contenir tout gaz inerte, tel que de l'azote ou de l'hélium. Il peut également contenir un gaz oxydant, en particulier de l'oxygène. Il peut aussi contenir de la vapeur d'eau. De préférence, le courant gazeux contient au moins un composé choisi parmi l'azote, l'oxygène et l'eau.

Les catalyseurs de type silicalite au titane auxquels s'applique le procédé de régénération selon l'invention sont des matériaux synthétiques cristallins de structure analogue à celle des zéolites, comprenant des oxydes de silicium et de titane et caractérisés par une bande d'absorption infrarouge à environ 950-960 cm⁻¹ Leur formule générale est typiquement

xTiO₂(1-x)SiO₂

dans laquelle x est compris entre 0,0001 et 0,5, de préférence entre 0,001 et 0,05.

Des matériaux de ce type, connus sous le nom de TS-1, présentent une structure zéolitique cristalline microporeuse analogue à celle de la zéolite ZSM-5. Les propriétés et les principales applications de ces composés sont connues (B Notari ; Structure-Activity and Selectivity Relationship in Heterogeneous Catalysis ; R.K. Grasselli and A.W. Sleight Editors ; Elsevier , 1991 ; p. 243-256). Leur synthèse a également été largement étudiée (A. Van der Poel et J. Van Hooff, Applied Catalysis A; 1992; volume 92, pages 93-111). D'autres matériaux de ce type ont une structure analogue à celle de la zéolite bêta, de la zéolite ZSM-11 ou de la zéolite MCM-41.

Le traitement thermique du catalyseur se fait généralement à une température d'au moins 150 °C. Une température de traitement d'au moins 175 °C est préférée. En présence d'une quantité substantielle d'eau dans le courant gazeux (au moins 0,01 % molaire), le procédé selon l'invention procure une excellente régénération à une température de 175 à 250 °C. La température est plus particulièrement de 180 à 220 °C. En l'absence substantielle de vapeur d'eau dans le courant gazeux, c'est-à-dire lorsque la quantité d'eau dans le courant gazeux est inférieure à 0,01 % molaire, de très bons résultats ont été obtenus à une température de 250 à 350 °C

La durée optimum du traitement thermique dépend de l'état de désactivation du catalyseur à régénérer. Elle est généralement d'au moins 30 minutes, de préférence d'au moins 1 heure. Le plus souvent, elle ne dépasse pas 20 heures. De bons résultats ont été obtenus avec une durée de traitement de 2 à 8 heures.

Le traitement thermique du catalyseur dans le procédé de régénération selon l'invention peut être réalisé par tout moyen adéquat réalisant un balayage du catalyseur par le courant gazeux. On peut par exemple effectuer le traitement dans un four rotatif équipé d'un système de balayage de gaz ou dans un réacteur à lit fixe ou à lit fluidisé.

Dans une forme d'exécution particulièrement préférée, le courant gazeux balayant le catalyseur contient de l'oxygène. Dans cette forme d'exécution, la teneur en oxygène dans le courant gazeux est d'au moins 1 % molaire. De préférence, elle est d'au moins 5 % molaire. Bien qu'en théorie, on puisse travailler avec un courant gazeux constitué essentiellement d'oxygène, on travaille le plus souvent avec un courant gazeux ne renfermant pas plus de 30 % molaire d'oxygène. Le solde du courant gazeux est alors constitué d'autres gaz, tels que de l'azote ou de la vapeur d'eau. D'excellents résultats ont été obtenus dans le cas où le courant gazeux est de l'air.

Dans une autre forme d'exécution particulièrement préférée, le courant gazeux balayant le catalyseur contient une quantité substantielle de vapeur d'eau. Dans cette forme d'exécution, la teneur en eau dans le courant gazeux est d'au moins 0,01 % molaire De préférence, elle est d'au moins 0,05 % molaire. On peut travailler avec un courant gazeux constitué essentiellement de vapeur d'eau. D'excellents résultats sont toutefois obtenus avec un courant gazeux renfermant moins de 10 % molaire d'eau. Le solde du courant gazeux est alors constitué d'autres gaz, tels que de l'azote ou de l'oxygène.

Dans une variante avantageuse de cette forme d'exécution préférée, le courant gazeux balayant le catalyseur contient de l'oxygène, en plus de la vapeur d'eau. Généralement, le courant gazeux est constitué essentiellement d'air humide contenant de 0,01 à 50 % molaire d'eau. De manière avantageuse, le courant gazeux est constitué essentiellement d'air humide contenant de 0,01 à 10 % molaire d'eau.

Dans une forme d'exécution du procédé de régénération selon l'invention, le traitement thermique est précédé d'un lavage du catalyseur avec de l'eau ou avec un composé organique (lequel est de préférence un alcool, le méthanol étant particulièrement préféré), afin d'éliminer substantiellement tous les composés avec lesquels le catalyseur a été en contact dans la réaction dans laquelle il a été mis en oeuvre. La température du liquide de lavage est généralement comprise entre 25 °C à la température d'ébullition du liquide de lavage. Ce lavage se fait par mise en contact du catalyseur avec de l'eau ou avec le composé organique durant une ou plusieurs périodes de 5 minutes à 2 heures.

Dans une forme d'exécution du procédé de régénération selon l'invention, le catalyseur est soumis, avant le traitement thermique, à un balayage avec un gaz inerte, le plus souvent de l'azote, à une température de 50 à 100 °C pendant une période de 10 minutes à 1 heure Le balayage du catalyseur a pour fonction d'éliminer les impuretés volatiles du catalyseur

En variante, le catalyseur peut être soumis au lavage et au balayage précités

Le procédé selon l'invention permet de restaurer de manière répétée quasi toute l'activité initiale du catalyseur

Le procédé selon l'invention s'applique aux catalyseurs de type silicalite au titane usagés, notamment ceux utilisés dans une réaction mettant en oeuvre du peroxyde d'hydrogène et un co-réactif organique, en particulier ceux utilisés dans des réactions d'époxydation d'oléfines au moyen de peroxyde d'hydrogène, d'hydroxylation de composés aromatiques ou d'oxydation d'hydrocarbures saturés. Il s'applique plus particulièrement aux catalyseurs utilisés dans des réactions d'époxydation d'oléfines, au moyen de peroxyde d'hydrogène Il s'applique tout particulièrement aux catalyseurs utilisés dans la réaction d'époxydation du chlorure d'allyle en épichlorhydrine au moyen de peroxyde d'hydrogène. En outre, le procédé peut être appliqué aux catalyseurs utilisés dans la réaction d'époxydation du propylène en oxyde de propylène au moyen de peroxyde d'hydrogène.

L'invention se trouve plus amplement illustrée dans les exemples non limitatifs suivants.

### Exemple 1

On a disposé dans un réacteur de 125 ml muni d'une boucle de recirculation (volume total = 250 ml), 7,7 grammes de catalyseur de type silicalite au titane TS-1. Le réacteur a été alimenté en continu à un débit de 250 ml/heure par une solution de chlorure d'allyle et de peroxyde d'hydrogène dans du méthanol (chlorure d'allyle/H₂O₂ = 2 mol/mol; concentration en H₂O₂ de 1,38 mol/kg) à la température de 36 °C. La vitesse linéaire de passage de la solution en recirculation dans le réacteur a été réglée à 1 m/min. La concentration en peroxyde d'hydrogène dans le mélange réactionnel soutiré a été mesurée par iodométrie. Dès que le taux de conversion du peroxyde d'hydrogène fut de 50 % inférieur à celui obtenu après une heure de marche, le réacteur a été vidangé Le catalyseur désactivé a été lavé par du méthanol circulant en boucle dans le réacteur à une température de 35 °C. Après purge du réacteur, le catalyseur désactivé a été transféré dans un tube en pyrex placé dans un four ventilé. Après avoir éliminé les composés volatils du catalyseur par un balayage de celui-ci par de l'azote à une température de 75 °C, on a soumis le catalyseur désactivé à un traitement thermique de régénération à une température de 300 °C, pendant 7 heures, sous un courant d'azote sec (teneur en eau inférieure ou égale à 6 mg/Nm³), à un débit de 200 lN/h.

Après refroidissement, le catalyseur régénéré a été disposé à nouveau dans le réacteur d'époxydation du chlorure d'allyle et le réacteur a été à nouveau alimenté par la solution de chlorure d'allyle et de peroxyde d'hydrogène dans le méthanol, dans les conditions exposées ci-dessus.

4 cycles tels que celui décrit ci-dessus d'utilisation/régénération du catalyseur ont été effectués. A chaque cycle, l'activité du catalyseur régénéré a éte mesurée en déterminant la quantité d'épichlorhydrine produite dans ces conditions avant que le taux de conversion du peroxyde d'hydrogène ne chute à nouveau de 25 % par rapport à sa valeur initiale mesurée après une heure de marche Des activités respectives de 95, 98, 94 et 94 grammes d'épichlorhydrine ont été mesurées lors de ces 4 cycles. A titre de comparaison, l'activité du catalyseur frais, c'est-à-dire lors de sa première utilisation, fut de 95 grammes d'épichlorhydrine.

### Exemple 2

11 cycles tels que celui décrit à l'exemple 1 ont été réalisés, en effectuant le traitement thermique de régénération du catalyseur désactivé, sous un courant d'azote contenant environ 25 g d'eau par Nm³, pendant 2,5 heures, toutes autres choses étant égales.

Au cours de ces onze cycles successifs d'utilisation/régénération du catalyseur TS-1, des activités respectives de 93, 97, 97, 95, 93, 94, 92, 93, 92, 91 et 94 grammes d'épichlorhydrine ont été mesurées.

### Exemple 3

8 cycles tels que celui décrit à l'exemple 1 ont été réalisés, en effectuant le traitement thermique de régénération du catalyseur désactivé, sous un courant d'air contenant environ 25 g d'eau par Nm³, pendant 7 heures (cycles 1 et 2), 15 heures (cycles 3 et 6) ou 5 heures (cycles 4, 5, 7 et 8), toutes autres choses étant égales.

Au cours de ces huit cycles successifs, des activités respectives de 96, 99, 98, 98, 96, 97, 101 et 98 grammes d'épichlorhydrine ont été mesurées.

### Exemple 4

3 cycles tels que celui décrit à l'exemple 1 ont été réalisés, en effectuant le traitement thermique de régénération du catalyseur désactivé à une température de 215 °C, sous un courant gazeux constitué d'azote et d'environ 700 g d'eau par Nm³, à un débit de 250 lN/h, pendant 3,5 heures, toutes autres choses étant égales

Au cours de ces 3 cycles successifs, des activités respectives de 94, 93 et 92 grammes d'épichlorhydrine ont été mesurées.

### Exemple 5

4 cycles tels que celui décrit à l'exemple 1 ont été réalisés, en effectuant le traitement thermique de régénération du catalyseur désactivé à une température de 200 °C, sous un courant d'air contenant 1 g d'eau par Nm³, toutes autres choses étant égales.

Au cours de ces 4 cycles successifs, des activités respectives de 91, 89, 91 et 91 grammes d'épichlorhydrine ont été mesurées.

### Exemple 6 (comparaison)

L'exemple 5 a été répété, en effectuant le traitement thermique de régénération du catalyseur désactivé sous un courant d'air contenant 1 g d'eau par Nm³, à un débit de 3 lN/h, toutes autres choses étant égales

Au cours des 4 cycles successifs, des activités respectives de 83, 80, 75 et 72 grammes d'épichlorhydrine ont été mesurées.

### Exemple 7

2 cycles tels que celui décrit à l'exemple 1 ont été réalisés, en effectuant le traitement thermique de régénération du catalyseur désactivé à une température de 155 °C, sous un courant d'oxygène contenant environ 25 g d'eau par Nm³, à un débit de 100 lN/h, toutes autres choses étant égales.

Au cours de ces 2 cycles successifs, des activités respectives de 84 et 85 grammes d'épichlorhydrine ont été mesurées.

### Exemple 8 (comparaison)

L'exemple 7 a été répété, en effectuant le traitement thermique de régénération du catalyseur désactivé à une température de 105 °C, toutes autres choses étant égales.

Au cours des deux cycles successifs, des activités respectives de 78 et 71 grammes d'épichlorhydrine ont été mesurées.

## Revendications

1. Procédé de régénération d'un catalyseur constitué de silicalite au titane issu de la synthèse d'un époxyde par réaction entre une oléfine et du peroxyde d'hydrogène comprenant un traitement thermique, **caractérisé en ce que**, durant le traitement thermique, on balaie le catalyseur, à une température d'au moins 130 °C, mais inférieure à 400 °C, par un courant gazeux dont le temps de séjour massique sur le catalyseur est d'au moins une minute et ne dépasse pas 1 heure.

2. Procédé selon la revendication 1, dans lequel le courant gazeux contient au moins un composé choisi parmi l'azote, l'oxygène et l'eau.

3. Procédé selon la revendication 1 ou 2, dans lequel le courant gazeux contient de l'oxygène.

4. Procédé selon l'une quelconque des revendications 1 à 3, dans lequel le courant gazeux contient au moins 0,01 % molaire d'eau et le traitement thermique s'effectue à une température de 175 à 250 °C.

5. Procédé selon la revendication 1, dans lequel le traitement se fait à une température de 150 à 350 °C.

6. Procédé selon l'une quelconque des revendications 1 a 5, dans lequel le traitement dure de 30 minutes à 8 heures.

7. Procédé selon l'une quelconque des revendications 1 à 6, appliqué à un catalyseur issu de la synthèse d'épichlorhydrine par réaction entre du chlorure d'allyle et du peroxyde d'hydrogène.

8. Procédé selon l'une quelconque des revendications 1 à 6, appliqué à un catalyseur issu de la synthèse d'oxyde de propylène par réaction entre du propylène et du peroxyde d'hydrogène.

9. Procédé de synthèse d'époxydes par réaction entre une oléfine et du peroxyde d'hydrogène en présence d'un catalyseur constitué de silicalite au titane, comprenant une étape de régénération du catalyseur par le procédé selon l'une quelconque des revendications 1 à 8.

10. Procédé selon la revendication 9, pour la synthèse d'épichlorhydrine par réaction entre du chlorure d'allyle et du peroxyde d'hydrogène.

11. Procédé selon la revendication 9, pour la synthèse d'oxyde de propylène par réaction entre du propylène et du peroxyde d'hydrogène.

## Patentansprüche

1. Verfahren zur Regenerierung eines aus Titansilicalit bestehenden Katalysators, der aus der Synthese eines Epoxids durch Umsetzung zwischen einem Olefin und Wasserstoffperoxid stammt, welches Verfahren eine Wärmebehandlung umfaßt, **dadurch gekennzeichnet, daß** während der Wärmebehandlung der Katalysator bei einer Temperatur von wenigstens 130°C, aber unter 400°C mit einem Gasstrom gespült wird, dessen Massenverweilzeit über dem Katalysator wenigstens eine Minute beträgt und eine Stunde nicht übersteigt.

2. Verfahren nach Anspruch 1, worin der Gasstrom wenigstens eine unter Stickstoff, Sauerstoff und Wasser ausgewählte Verbindung enthält.

3. Verfahren nach Anspruch 1 oder 2, worin der Gasstrom Sauerstoff enthält.

4. Verfahren nach einem der Ansprüche 1 bis 3, worin der Gasstrom wenigstens 0,01 Mol-% Wasser enthält und die Wärmebehandlung bei einer Temperatur von 175 bis 250°C vorgenommen wird.

5. Verfahren nach Anspruch 1, worin die Behandlung bei einer Temperatur von 150 bis 350°C erfolgt.

6. Verfahren nach einem der Ansprüche 1 bis 5, worin die Behandlung 30 Minuten bis 8 Stunden dauert.

7. Verfahren nach einem der Ansprüche 1 bis 6, angewendet auf einen Katalysator, der aus der Epichlorhydrinsynthese durch Umsetzung von Allylchlorid mit Wasserstoffperoxid stammt.

8. Verfahren nach einem der Ansprüche 1 bis 6, angewendet auf einen Katalysator, der aus der Propylenoxidsynthese durch Umsetzung von Propylen mit Wasserstoffperoxid stammt.

9. Verfahren zur Synthese von Epoxiden durch Umsetzung zwischen einem Olefin und Wasserstoffperoxid in Gegenwart eines aus Titansilicalit bestehenden Katalysators, umfassend eine Regenerationsstufe des Katalysators nach dem Verfahren gemäß einem der Ansprüche 1 bis 8.

10. Verfahren nach Anspruch 9 für die Synthese von Epichlorhydrin durch Umsetzung von Allylchlorid mit Wasserstoffperoxid.

11. Verfahren nach Anspruch 9 für die Synthese von Propylenoxid durch Umsetzung von Propylen mit Wasserstoffperoxid.

## Claims

1. Process for the regeneration of a catalyst composed of titanium silicalite, resulting from the synthesis of an epoxide by reaction between an olefin and hydrogen peroxide, comprising a heat treatment, **characterized in that**, during the heat treatment, the catalyst is stripped at a temperature of at least 130°C but less than 400°C by a gas stream, the mass residence time of which on the catalyst is at least one minute and does not exceed 1 hour.

2. Process according to Claim 1, in which the gas stream contains at least one compound chosen from nitrogen, oxygen and water.

3. Process according to Claim 1 or 2, in which the gas stream contains oxygen.

4. Process according to any one of Claims 1 to 3, in which the gas stream contains at least 0.01 molar % of water and the heat treatment is carried out at a temperature of 175 to 250°C.

5. Process according to Claim 1, in which the treatment is carried out at a temperature of 150 to 350°C.

6. Process according to any one of Claims 1 to 5, in which the treatment lasts from 30 minutes to 8 hours.

7. Process according to any one of Claims 1 to 6, applied to a catalyst resulting from the synthesis of epichlorohydrin by reaction between allyl chloride and hydrogen peroxide.

8. Process according to any one of Claims 1 to 6, applied to a catalyst resulting from the synthesis of propylene oxide by reaction between propylene and hydrogen peroxide.

9. Process for the synthesis of epoxides by reaction between an olefin and hydrogen peroxide in the presence of a catalyst composed of titanium silicalite, comprising a stage of regeneration of the catalyst by the process according to any one of Claims 1 to 8.

10. Process according to Claim 9, for the synthesis of epichlorohydrin by reaction between allyl chloride and hydrogen peroxide.

11. Process according to Claim 9, for the synthesis of propylene oxide by reaction between propylene and hydrogen peroxide.
